(19) Europäisches Patentamt
European Patent Office
Office européen des brevets

(11) Veröffentlichungsnummer: **0 210 564**
**B1**

(12) # EUROPÄISCHE PATENTSCHRIFT

(45) Veröffentlichungstag der Patentschrift: **04.04.90**

(21) Anmeldenummer: **86109959.6**

(22) Anmeldetag: **21.07.86**

(51) Int. Cl.[5]: **C 07 C 37/00,** C 07 C 37/055, C 07 C 39/16, C 07 C 49/83, C 07 C 49/84

(54) **Verfahren zur Herstellung von Bis-hydroxyphenyl-n-alkanen.**

(30) Priorität: **02.08.85 DE 3527862**

(43) Veröffentlichungstag der Anmeldung:
**04.02.87 Patentblatt 87/06**

(45) Bekanntmachung des Hinweises auf die Patenterteilung:
**04.04.90 Patentblatt 90/14**

(84) Benannte Vertragsstaaten:
**DE FR GB NL**

(56) Entgegenhaltungen:
**EP-A-0 157 740**
**DE-A-2 139 994**
**DE-B- 817 459**

**SYNTHESIS, Nr. 9, September 1978, pages 672-675, Georg Thieme Publishers; G.A. OLAH et al.: "Heterogenous Catalysis by solid superacids; 11 1. Perfluorinated resinsulfonic acid (Nafion-H)2 catalyzed friedel-crafts acylation of benzene and substituted benzenes"**

(73) Patentinhaber: **BAYER AG**
**D-5090 Leverkusen 1 Bayerwerk (DE)**

(72) Erfinder: **Wagner, Rudolf, Dr.**
**Hahnenweg 4**
**D-5000 Köln 80 (DE)**
Erfinder: **Halcour, Kurt, Dr.**
**Gellertstrasse 14**
**D-5090 Leverkusen 1 (DE)**
Erfinder: **Dicke, Hans-Rudolf, Dr.**
**Bodelschwinghstrasse 16**
**D-4150 Krefeld (DE)**
Erfinder: **Eckhardt, Volker, Dr.**
**Bodelschwinghstrasse 14**
**D-4150 Krefeld (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 210 564 B1

**Beschreibung**

Die vorliegende Erfindung betrifft ein Verfahren zur Herstellung von Bis-hydroxyphenyl-n-alkanen, indem man Dicarbonsäurederivate mit Fluor enthaltenden organischen Sulfonsäuren zusammenbringt und die so erhältlichen Alkandione durch Hydrierung und gegebenenfalls durch eine zusätzliche Etherspaltung in Bis-hydroxyphenyl-n-alkane überführt.

Die Herstellung einiger bestimmter Alkandione ist bereits aus Journal Indian Chem. Soc. *46*, No. 4, 351—357 und *46*, No. 8, 743—746 (1969) bekannt. Dabei werden Dichloride der Pimelin-, Kork-, Azelain- oder Sebacinsäure mit Phenol oder Anisol unter Zusatz von Aluminiumchlorid umgestzt. Dabei werden allerdings nur unbefriedrigende Ausbeuten und Selektivitäten erzielt. Ferner kann das eingesetzte Aluminiumchlorid nicht wiedergewonnen und erneut verwendet werden.

Aus der DE—A 2139994 ist bekannt, aromatische und heterocyclische Ketone in Gegenwart von Perfluoralkansulfonsäuren herzustellen.

Auch perfluorierte Alkylsulfonsäureharze wurden zur Herstellung von Ketonen verwendet (z.B. Synthesis 1978, S. 672, 673).

Auch die Herstellung bestimmter Bis-hydroxyphenyl-n-alkane ist bereits bekannt (siehe Zh. Organ. Khim. 1 (9), 1602—1604 (1965) und C.A. *64*, 623 f). Dabei werden Säurechloride von Adipin-, Kork- oder Sebacinsäure mit Anisol und Aluminiumchlorid umgesetzt, anschließend die erhaltenen Dione mit Hydrazin reduziert und anschließend daran eine Demethylierung mit Jodwasserstoffsäure durchgeführt. Dieses Verfahren hat die gleichen Nachteile wie das zuvor geschilderte.

Es wurde nun ein Verfahren zur Hertellung von Bis-hydroxyphenyl-n-alkanen der Formel (I)

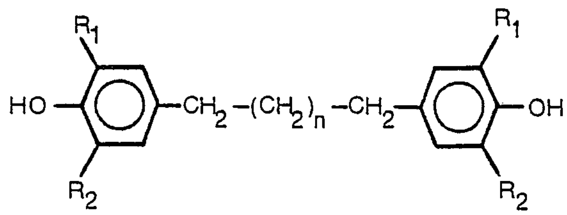

(I)

in der

n für eine ganze Zahl im Bereich von 0 bis 10 steht und

$R_1$ und $R_2$ unabhängig voneinander für Wasserstoff, $C_1$- bis $C_{12}$-Alkyl, $C_1$- bis $C_4$-Alkoxy und/oder Halogen stehen, gefunden, das dadurch gekennzeichnet ist, daß man Dicarbonsäurederivate der Formel (II)

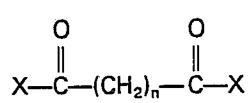

(II)

in der

n die oben angegebene Bedeutung hat und

für den Rest (III) steht

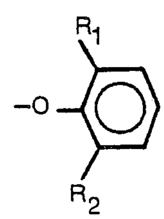

(III)

in dem $R_1$ und $R_2$ die oben angegebene Bedeutung haben, mit Fluor enthaltenden organischen Sulfonsäuren zusammenbrigt, und so Alkandione der Formel (IV) erhält

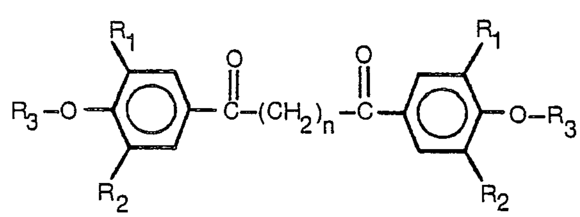

(IV)

in der

n, $R_1$, $R_2$ die oben angegebene Bedeutung haben und

$R_3$ für Wasserstoff steht, die man durch Hydrierung in die Bis-hydroxyphenyl-n-alkane der Formel (I) überführt.

In den Formeln (I), (II) und (IV) steht n vorzugsweise für eine ganze Zahl im Bereich von 6 bis 10. In den Formeln (I), (III), und (IV) stehen $R_1$ und $R_2$ vorzugsweise unabhängig voneinander für Wasserstoff, $C_1$- bis $C_6$-Alkyl, Fluor, Chlor und/oder Brom. Besonders bevorzugt sind $R_1$ und $R_2$ identisch und stehen für Wasserstoff, Methyl, Ethyl oder Chlor. Ganz besonders bevorzugt stehen $R_1$ und $R_2$ für Wasserstoff.

In der Formel (IV) steht $R_3$ für Wasserstoff. $R_1$ und $R_2$ können, soweit es sich jeweils um Halogen handelt, Fluor, Chlor, Brom und/oder lod bedeuten. Bevorzugt ist dabei Fluor, Chlor und Brom, besonders bevorzugt Chlor.

Bei den in das erfindungsgemäße Verfahren einzusetzenden Verbindungen der Formel (II) handelt es sich um Diphenylester von gesättigten aliphatischen Dicarbonsäuro mit 2 bis 12 C-Atomen. Beispiele für solche Dicarbonsäure diphenylester sind Oxal-, Malon-, Bernstein-, Glutar-, Adipin-, Pimelin-, Kork-, Azelain-, Sebacin-, Nonandicarbon- und Decandicarbonsäure diphenylester. Alle diese Verbindungen sind Handelsprodukte oder auf einfache Weise nach üblichen Methoden herstellbar.

Bei den in das erfindungsgemäße Verfahren einsetzbaren Verbindungen der Formel (II) sind die Diphenylester der Dicarbonsäuren die im Phenylteil in 2- und/oder 6-Stellung durch $C_1$- bis $C_{12}$-Alkyl, $C_1$- bis $C_4$-Alkoxy und/oder Halogen substituiert sein können. Beispiele für solche Verbindungen sind Glutarsäurediphenylester, Adipinsäurediphenylester, Korksäurediphenylester, Azelainsäure diphenylester, Sebacinsäurediphenylester, Decandicarbonsäurediphenylester und Sebacinsäure-bis-(2,6-dimethylphenyl)-ester. Derartige Diphenylester können beispielsweise durch die Umsetzung von Diphenylcarbonaten der Formel (VII)

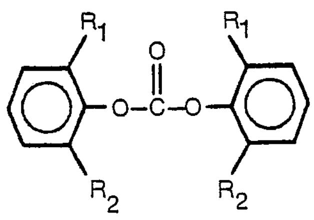

(VII)

in der

$R_1$ und $R_2$ die oben angegebene Bedeutung haben, oder durch die Umsetzung von Phenolen der Formel (VIII)

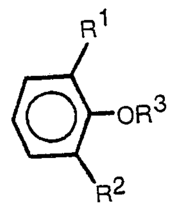

(VIII),

in welcher

$R_1$ und $R_2$ die oben angegebene Bedeutung haben und

$R_3$ für Wasserstoff steht,

jeweils mit Dicarbonsäuren oder Dicarbonsäurederivaten der abgewandelten Formel (II), in der X für OH oder Halogen steht oder beide X den Molekülteil $—CO—(CH_2)_n—CO—$ zu dem entsprechenden cyclischen oder polymeren Dicarbonsäureanhydrid ergänzen, in Gegenwart basischer Katalysatoren erhalten werden.

Es ist ein wesentliches Merkmal des erfindungsgemäßen Verfahrens, daß die Verbindungen der Formel (II), in den beschriebenen Fällen unter Zusatz von Verbindungen der Formel (IV), mit Fluor enthaltenden organischen Sulfonsäuren zusammengebracht werden. Bei den Fluor enthaltenden organischen Sulfonsäuren kann es sich bespielsweise um vollständig oder teilweise mit Fluoratomen substituierte Alkansulfonsäuren, um vollständig mit Fluor substituierte aromatische Sulfonsäuren oder um entsprechende fluorhaltige Sulfonsäurereste handeln, die an eine Harzmatrix gebunden sind. Bevorzugt sind organische Sulfonsäuren, die am C- Atom, das der Sulfonsäuregruppe benachbart ist, 1 bis 3 Fluoratome enthalten. Besonders bevorzugt sind perfluorierte Alkansulfonsäuren mit beispielsweise 2 bis 8 C-Atomen und entsprechende Sulfonsäurereste enthaltende Harzmatrices, insbesondere Trifluormethansulfonsäure, Perfluorbutansulfonsäure, Perfluoroctansulfonsäure und Ionenaustauscher, die unter der Bezeichnung Nafion® im Handel sind.

Die Fluor enthaltenden organischen Sulfonsäuren können beispielsweise in Mengen von 1 bis 10 Mol pro Mol der Verbindung der Formel (II) eingesetzt werden. Vorzugsweise beträgt diese Menge 1,5 bis 2,5 Mol, besonders bevorzugt 1,9 bis 2,1 Mol Fluor enthaltende organische Sulfonsäure pro Mol der Verbindung der Formel (II).

Als Temperaturen für die Bildung der Alkandione der Formel (IV) können beispeilsweise solche im Bereich von 20 bis 120°C eingehalten werden. Vorzugsweise hält man hierbei die Temperatur im Bereich von 20 bis 60°C. Üblicherweise arbeitet man hierbei bei Normaldruck. Es kann jedoch auch bei vermindertem oder erhöhtem Druck gearbeitet werden, beispeilsweise im Bereich von 0,5 bis 5 bar. Die Gegenwart von Lösungsmitteln ist nicht erforderlich, aber möglich und manchmal vorteilhaft. Geeignete

Lösungsmittel sind z.B. solche, die aprotisch und gegen Säuren beständig sind. Bevorzugt sind chlorierte gesättigte Kohlenwasserstoff, wie Methylenchlorid, Chloroform und Dichlorethan.

Die Verweilzeiten für die Bildung der Alkandione der Formel (IV) können in weiten Grenzen variiert werden. Beispielsweise sind Verweilzeiten im Bereich von 1 Minute bis 2 Stunden geeignet. Bevorzugt sind Verweilzeiten im Bereich von 20 bis 60 Minuten.

Die Aufarbeitung des danach vorliegenden Reaktionsgemisches kann beispielsweise so durchgeführt werden, daß man, sofern ein Lösungsmittel vorhanden ist, dieses zunächst entfernt, beispielsweise durch Destillation, gegebenenfalls unter vermindertem Druck. Es ist ein besonderer Vorteil des erfindungsgemäßen Verfahrens, daß man die Fluorenthaltende organische Sulfonsäure zurüchgewinnen und erneut einsetzen kann. Hierzu kann man beispielsweise das, gegebenenfalls vom Lösungsmittel befreite, Reaktionsgemisch mit Wasser versetzen. Dabei fällt das Reaktionsprodukt (die Verbindung der Formel (IV) aus. Es kann abfiltriert und, falls gewünscht, beispielsweise durch Imkristallisation zusätzlich gereinigt werden. Eine solche Umkristallisation kann beispielsweise mit Alkoholen, Alkohol-Wasser-Gemischen oder aromatischen Kohlenwasserstoffen durchgeführt werden. Bevorzugt sind dabei Ethanol, Ethanol-Wasser-Gemische und Toluol. Das wäßrige Filtrat enthält die eingesetzte, Fluor enthaltende organische Sulfonsäure. Zu deren Rückgewinnung kann man, gegebenenfalls nach einer Wäsche mit einem organischen Lösungsmittel, z.B. Ethylacetate, eine zur Bildung unlöslicher Salze befähigter Base zufügen, beispielsweise ein Alkali- oder Erdalkalihydroxid, insbesondere Kalium-, Calcium- oder Bariumhydroxid. Das Salz der Fluor enthaltenden organischen Sulfonsäure kann dann, vorzugsweise nach einer Trocknung, mit hochkonzentrierter, z.B. 100%-iger Schwefelsäure versetzt und aus diesem Gemisch durch Destillation bei vermindertem Druck die Fluor enthaltende organische Sulfonsäure gewonnen und erneut eingesetzt werden. Bie dieser Arbeitsweise sind Rückgewinnungsraten von beispielsweise über 95% zu erzielen.

Die Abtrennung von an einer Harzmatrix gebundenen Fluor enthaltenden organischen Sulfonsäure kann durch einfaches Filtrieren des Reaktionsgemisches erfolgen. Sie ist, gegebenenfalls nach Waschen und Trocknen, wieder einsetzbar. Aus dem Filtrat kann dann, wie oben beschrieben, die Verbindung der Formel (IV) isoliert werden.

Die Hydrierung der erfindungsgemäß hergestellten Verbindungen der Formel (IV) zu den Bis-hydroxyphenyl-n-alkanen der Formel (I) kann auf an sich bekannter Weise erfolgen. Man kann beispielsweise die Verbindung der Formel (IV) in einem Lösungsmittel auflösen, geeignet sind beispielsweise Tetrahydrofuran und Dioxan, und bei 60 bis 150°C, vorzugsweise 80 bis 120°C bei einem Wasserstoffpartialdruck von 5 bis 50 bar, vorzugsweise 15 bis 25 bar, und einer Verweilzeit von 0,1 bis 4 Stunden, vorzugsweise 0,5 bis 2 Stunden, katalytisch hydrieren. Als Katalysatoren eignen sich solche mit oder ohne Träger, die bekanntermaßen eine selektive Hydrierung von >C = O-Gruppen zu $>CH_2$-Gruppen ermöglichen. Bevorzugt werden Nickel oder Palladium enthaltende Katalysaturen, insbesondere Palladium enthaltende verwendet. Aus dem nach der Hydrierung vorliegende Reaktionsgemisch kann das Hydrierprodukt beispielsweise gewonnen werden, indem man den Katalysator durch Filtration und das Lösungsmittel durch Destillation abtrennt. Das zurückbleibende Hydrierprodukt kann gegebenenfalls durch Umkristallisation, beispielsweise aus aromatischen Kohlenwasserstoffen, gereinigt werden.

Es ist ausgesprochen überraschend, daß es mit den erfindungsgemäß einzusetzenden Fluor enthaltenden organischen Sulfonsäuren gelingt, Verbindungen der Formel (I) in höheren Ausbeuten und besseren Selektivitäten zu erhalten, als beim bekannten Einsatz von Aluminiumchlorid. Beachtenswert ist ferner, daß die erfindungsgamäß einzusetzenden Säuren, im Gegensatz zu Aluminiumchlorid, wiedergewonnen und erneut verwendet werden können, was das erfindungsgemäße Verfahren besonders umweltfreundlich und wirdschaftlich macht. Überraschend ist ferner, daß die Fluor enthaltenden organischen Sulfonsäuren eine gute Para-Selektivität aufweisen, d.h. selektiv Produkte der Formel (I) erhalten werden, bei denen sich die phenolische OH-Gruppe und die $CH_2$-Kette in Para-Stellung am jeweiligen aromatischen Ring befinden. Aus der Tatsache, daß sich der Verknüpfungspunkt des phenolischen Molekülteils mit dem Restmolekül verändert ist ersichtlich, daß der phenolische Molekülteil unter den Reaktionsbedingungen des erfindungsgemäßen Verfahrens beweglich ist (siehe das folgende Reaktionsschema)

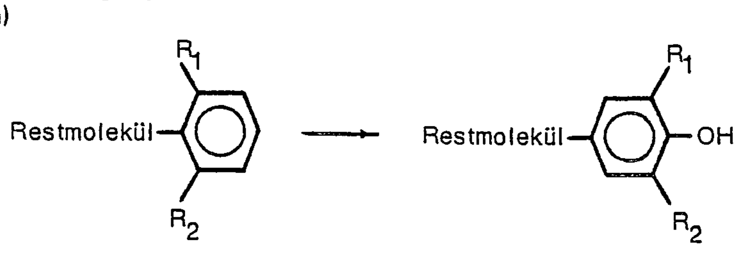

und deshalb eine gute Para-Selektivität nicht zu erwarten war.

Die erfindungsgemäß herstellbaren Bis-hydroxyphenyl-n-alkane der Formel (I) können als Ausgangssubstanzen zur Herstellung von Farbstoffen, Wirkstoffen und Polykondensaten, beispielsweise Polyestern und Polyethern, verwendet werden. Weiterhin besitzen diese Verbindungen Bedeutung als Additive zur Stabilisierung von thermoplastischen Werkstoffen, beispielsweise solchen die in der DE—OS 3 502 378 beschrieben sind.

Beispiel 1

52 g (0,2 mol) Gutarsäurediphenylester, 120 g (0,4 mol) Perfluorbutansulfonsäure und 200 ml 1,2-Dichlorethan wurden unter Feuchtigkeitsbeschluß zusammengegeben und 2 Stunden bei Raumtemperatur gerührt. Anschließend wurde das Methylenchlorid bei einer Badtemperatur von 30°C im Vakuum verdampft. Der rote Rückstand wurde in 500 ml Wasser gegossen und der Niederschlag abfiltriert. 1 g des so erhaltenen Produktes wurde per Saülenchromatographie auf Kieselgel mit 15% Ethylacetat in Hexan aufgearbeitet, 1,5-Bis- (4-hydroxyphenyl)-1,5-pentandion (0,2 g) wurde als rotes Öl in der 2, eluierten Komponente gewonnen und massenspektroskopisch ($M^+$284) und mittels $^1H$—NMR-Spektroskopie (in $d_6$-DMSO δ 7,86 (4H, d), δ 6,87 (4H, d), δ 3,04 (4H, t), δ 2,15 (2H, m), δ 8,7 (2H, s)) charakterisiert. Insgesamt wurde 1,5-Bis(4-hydroxyphenyl)-1,5-pentandion in einer Ausbeute von 17 g (30% der Theorie) und mit einem Schmelzpunkt von 158 bis 163°C (Zersetzung) erhalten.

Beispiel 2

Es wurde eine Umsetzung durchgeführt wie in Beispiel 1 beschrieben, jedoch wurde anstelle von Glutarsäurediphenylester 54,8 g (0,2 mol) Adipinsäurediphenylsäureester eingesetzt. Nach der Entfernung des Lösungsmittels wurde das Reaktionsgemisch in 500 ml Wasser gegossen und der sich bildende gelbe Niederschlag abfiltriert. Der Filtrationsrückstand wurde aus Ethanol/Wasser umkristallisiert. Es wurden 33 g (60% der Theorie) 1,6-Bis-(4-hydroxyphenyl)-1,6-hexandion als hellgelber Feststoff mit einem Schmelzpunkt von 232 bis 238°C (Zersetzung) erhalten.

Beispiel 3

Es wurde verfahren zie in Beispiel 2, jedoch wurde anstelle von Adipinsäurediphenylester 65,2 g (0,2 mol) Korksäurediphenylester eingesetzt und bei 80°C (anstelle Raumtemperatur) gerührt. Es wurden 54,1 g (83% der Theorie) 1,8-Bis-(4-hydroxyphenyl)-1,8-octandion als hellgelbar Feststoff mit einem Schmelzpunkt von 196 bis 198°C erhalten.

Beispiel 4

Es wurde verfahren wien in Beispiel 2 beschrieben, jedoch wurde anstelle von Adipinsäurediphenylester 68 g (0,2 mol) Azelainsäurediphenylester eingesetzt und zur Umkristallisation des Produktes Toluol verwendet. Es wurden 54,4 g (80% der Theorie) 1,9-Bis-(4-hydroxyphenyl)-1,9-nonandion als hellgelber Feststoff mit einem Schmelzpunkt von 130 bis 137°C (Zersetzung) erhalten.

Beispiel 5

Es wurde verfahren wie in Beispiel 3 beschrieben, jedoch wurde anstelle von Korksäurediphenylester 70,8 g (0,2 mol) Sebacinsäurediphenylester eingesetzt. Es wurden 53,8 g (76% der Theorie) 1,10-Bis-(4-hydroxyphenyl)-1,10-decandion mit einem Schmelzpunkt von 193 bis 194°C (Zersetzung) erhalten.

Beispiel 6

Es wurde verfahren wie in Beispiel 3 beschrieben, jedoch wurde anstelle von Korksäurediphenylester 76,4 g (0,2 mol) Dodecandisäurediphenylester eingesetzt. Es wurden 45,8 g (60% der Theorie) α,ω-Bis-(4-hydroxyphenyl)-α,ω-dodecandion als hellgelber Feststoff mit einem Schmelzpunkt von 177 bis 181°C (Zersetzung) erhalten.

Beispiel 7

Jeweils 100 g Dihydroxydiketon wurden in 400 ml Dioxan gelöst und mit 5 g 5 %igem Palladium auf Kohle versetzt. Danach wurde in einem 700 ml fassenden Rührautoklav bei 100°C und einem Wasserstoffdruck von 20 bar hydriert. Danach wurde der Katalysator abfiltriert, das Dioxan abdestilliert und der Rückstand aus 300 ml Toluol umkristallisiert. Die eingesetzten Dihydroxydiketone, die erhaltenen Bis-(4-hydroxyphenyl)-n-alkane, die Ausbeuten und Schmelzpunkte sind aus Tabelle 1 ersichtlich.

TABELLE 1

| eingesetztes Dihydroxydiketon (1,x-Bis-(4-hydroxyphenyl)-1,x-y-dion) | erhaltenes Hydrierprodukt (1,x-Bis-(4-hydroxyphenyl)-n-y) | Ausbeute (g) | (% der Theorie) | Schmelzpunkt (°C) |
|---|---|---|---|---|
| x = 6, y = hexan | x = 6, y = hexan | 77 | 85 | 143—144 |
| x = 8, y = octan | x = 8, y = octan | 73 | 80 | 138—139 |
| x = 10, y = decan | x = 10, y = decan | 76 | 83 | 138—139 |
| x = 12, y = dodecan | x = 12, y = dodecan | 70 | 75 | 134—135 |

**Patentansprüche**

1. Verfahren zur Herstellung von Bis-hydroxyphenyl-n-alkanen der Formel (I)

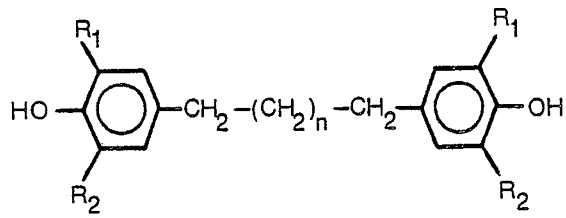

(I)

in der

n für eine ganze Zahl im Bereich von 0 bis 10 steht und

$R_1$ und $R_2$ unabhängig voneinander für Wasserstoff, $C_1$- bis $C_{12}$-Alkyl, $C_1$- bis $C_4$-Alkoxy und/oder Halogen stehen, dadurch gekennzeichnet, daß man Dicarbonsäurederivate der Formel (II)

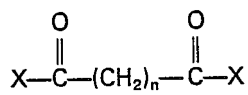

(II)

in der

n die oben angegebene Bedeutung hat und

für den Rest (III) steht

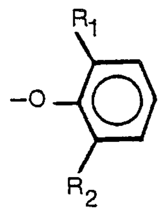

(III)

in dem $R_1$ und $R_2$ die oben angegebene Bedeutung haben, mit Fluor enthaltenden organischen Sulfonsäuren zusammenbringt, und so Alkandione der formel (IV) erhält

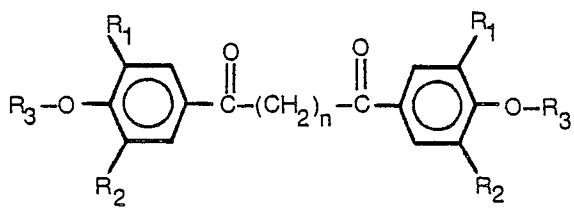

(IV)

in der

n, $R_1$, $R_2$ die oben angegebene Bedeutung haben und

$R_3$ für Wasserstoff steht, die man durch Hydrierung in die Bis-hydroxyphenyl-n-alkane der Formel (I) überführt.

2. Verfahren nach Anspruch 1, dadurch gekennzeichnet, daß man als Fluor enthaltende organische Sulfonsäure eine mit Fluoratomen substituierte Alkansulfonsäure oder einen entsprechenden fluorhaltigen Sulfonsäurerest, der an eine Harzmatrix gebunden ist, einsetzt.

3. Verfahren nach Anspruch 2, dadurch gekennzeichnet, daß man eine perfluorierte Alkansulfonsäure mit 2 bis 8 C-Atomen oder entsprechende Sulfonsäurereste enthaltende Harzmatrices einsetzt.

4. Verfahren nach Ansprüchen 1 bis 3, dadurch gekennzeichnet, daß man Fluor enthaltende organische Sulfonsäure in einer Menge von 1 bis 10 Mol pro Mol der Verbindung der Formel (II) einsetzt.

5. Verfahren nach Anspruch 4, dadurch gekennzeichnet, daß man auf ein Mol einer Verbindung der Formel (II) 1,5 bis 2,5 Mol Fluor enthaltende organische Sulfonsäure einsetzt.

6. Verfahren nach Ansprüchen 1 bis 5, dadurch gekennzeichnet, daß man bei der Bildung der Alkandione der Formel (IV) Temperaturen im Bereich von 20 bis 120°C, Drucke im Bereich von 0,5 bis 5 bar und Verweilzeiten im Bereich von 1 Minute bis 2 Stunden einhält.

**Revendications**

1. Procédé de production de bis-hydroxyphényl-n-alcanes de formule (I)

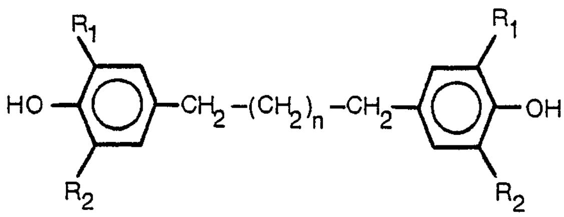

(I)

dans laquelle

n est un nombre entier compris entre 0 à 10 et

$R_1$ et $R_2$ représentent, indépendamment l'un de l'autre, l'hydrogène, un groupe alkyle en $C_1$ à $C_{12}$, alkoxy en $C_1$ à $C_4$ et/ou un halogène, caractérisé en ce qu'on met en présence des dérivés d'acides dicarboxyliques de formule (II)

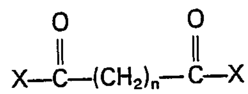

(II)

dans laquelle

n a la définition donné ci-dessus et

X représente le reste (III)

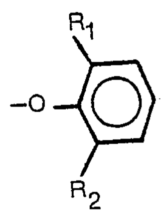

(III)

dans lequel $R_1$ et $R_2$ ont la définiton indiquée ci-dessus, et des acides sulfoniques organiques contenant du fluor, et on obtient ainsi des alcanediones de formule (IV)

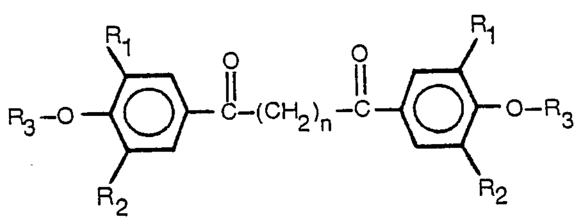

(IV)

dans laquelle

n, $R_1$, $R_2$ ont la définition indiquée ci-dessus et $R_3$ représente l'hydrogène, que l'on tranforme par hydrogénation en les bis-hydroxyphényl-n-alcanes de formule (I).

2. Procédé suivant la revendication 1, caractérisé en ce qu'on utilise comme acide sulfonique organique contenant du fluor un acide alcanesulfonique substitué avec des atomes de fluor ou un reste d'acide sulfonique fluoré correspondant, qui est lié à une matrice de résine.

3. Procédé suivant la revendication 2, caractérisé en ce qu'on utilise un acide alcanesulfonique perfluoré ayant 2 à 8 atomes de carbone ou des matrices de résine contenant des restes d'acide sulfonique correspondants.

4. Procédé suivant les revendications 1 à 3, caractérisé en ce qu'on utilise l'acide sulfonique organique contenant du fluor en une quantité de 1 à 10 moles par mole de composé de formule (II).

5. Procédé suivant la revendication 4, caractérisé en ce qu'on utilise, par mole d'un composé de formule (II), 1,5 à 2,5 moles d'acide sulfonique organique contenant du fluor.

6. Procédé suivant les revendications 1 à 5, caractérisé en ce qu'on maintient des températures dans la plage de 20 à 120°C, des pressions dans la plage de 0,5 à 5 bars et des durées de séjour allant d'une minute à 2 heures au cours de la formation des alcanediones de formule (IV).

**Claims**

1. A process for the production of bis-hydroxyphenyl-n-alkanes corresponding to formula (I)

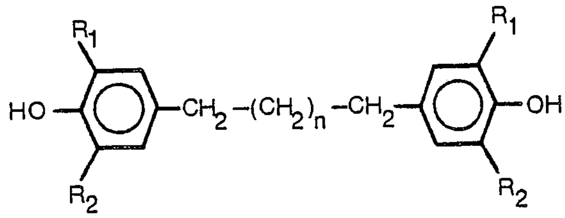

(I)

in which
n is an integer of 0 to 10 and
$R_1$ and $R_2$ independently of one another represent hydrogen, $C_{1-12}$ alkyl, $C_{1-4}$ alkoxy and/or halogen, characterized in that dicarboxylic acid derivatives corresponding to formula (II)

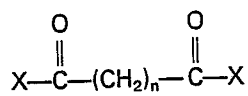

(II)

in which
n is as defined above and
X represents the group (III)

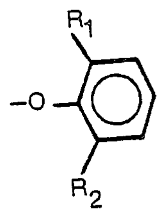

(III)

in which
$R_1$ and $R_2$ are as defined above, are combined with fluorine-containing organic sulfonic acids, giving alkanediones corresponding to formula (IV)

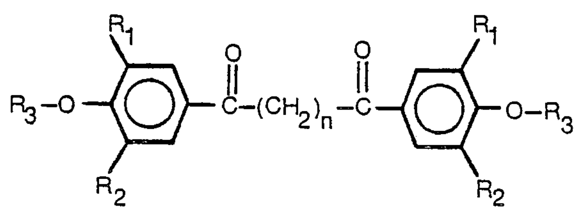

(IV)

in which
n, $R_1$, $R_2$ are as defined above and $R_3$ is hydrogen, which are then converted into the bis-hydroxyphenyl n-alkanes corresponding to formula (I).

2. A process as claimed in claim 1, characterized in that an alkanesulfonic acid substituted by fluorine atoms or a corresponding fluorine-containing sulfonic acid residue attached to a resin matrix is used as the fluorine-containing organic sulfonic acid.

3. A process as claimed in claim 2, characterized in that a perfluorinated alkanesulfonic acid containing 2 to 8 carbon atoms or resin matrices containing corresponding sulfonic acid residues is/are used.

4. A process as claimed in claims 1 to 3, characterized in that the fluorine-containing sulfonic acid is used in a quantity of from 1 to 10 mol per mol of the compound corresponding to formula (III).

5. A process as claimed in claim 4, characterized in that 1.5 to 2.5 mol fluorine-containing sulfonic acid are used per mol compound corresponding to formula (II).

6. A process as claimed in claims 1 to 5, characterized in that temperatures in the range from 20 to 120°C, pressures in the range from 0.5 to 5 bar and residence times of 1 minute to 2 hours are maintained during the formation of the alkanediones corresponding to formula (IV).